# FASCICULE DE BREVET EUROPEEN

(11) **EP 1 875 881 B1**
(45) Date de publication et mention de la délivrance du brevet: **02.03.2016**
(21) Numéro de dépôt: 07354039.5
(22) Date de dépôt: 02.07.2007
(51) Int. Cl.: A61F 2/80

(54) **DISPOSITIF DE JONCTION PAR EMBOÎTEMENT ÉTANCHE ENTRE UNE PROTHÈSE ET UN MANCHON INSTALLÉSUR LE MOIGNON D'UN MEMBRE AMPUTÉ, ET PROTHÈSE ÉQUIPÉE D'UN TEL DISPOSITIF DE JONCTION**
VERBINDUNGSVORRICHTUNG ZUR HERSTELLUNG EINER DICHTEN STECKVERBINDUNG ZWISCHEN EINER PROTHESE UND EINER HALTERUNG, DIE AUF DEM STUMPF EINES AMPUTIERTEN GLIEDS ANGEBRACHT IST, SOWIE PROTHESE, DIE MIT EINER SOLCHEN VERBINDUNGSVORRICHTUNG AUSGESTATTET IST.
JOINING DEVICE BY WATERTIGHT FITTING BETWEEN A PROSTHESIS AND A SLEEVE INSTALLED ON THE STUMP OF AN AMPUTATED LIMB, AND PROSTHESIS EQUIPPED WITH SUCH A JOINING DEVICE

(30) Priorité: 06.07.2006 FR 0606157
(43) Date de publication de la demande: 09.01.2008
(73) Titulaire: Chabloz Composants, 38170 Seyssinet-Pariset (FR)
(72) Inventeur: Chabloz, Pierre, 38450 Saint-Georges-de-Commiers (FR)
(74) Mandataire: Hecké, Gérard

(56) Documents cités:
- EP-A1- 0 631 765
- DE-C- 605 534
- DE-C- 746 781
- DE-C- 808 975
- DE-U- 1 795 809
- US-A1- 2 569 790
- US-A1- 2 834 025
- US-A1- 4 595 172

## Description

### Domaine technique de l'invention.

La présente invention est du domaine des prothèses non implantables dans le corps destinées à remplacer une partie manquante d'un membre amputé, tel qu'un bras ou une jambe par exemple. Elle a pour objet un dispositif de jonction par emboîtement étanche entre une prothèse et un manchon installé sur le moignon du membre amputé, ainsi qu'une prothèse équipée d'un tel dispositif de jonction.

### Etat de la technique.

Dans le domaine des prothèses, on connaît celles destinées à remplacer une partie manquante d'un membre amputé, tel qu'un bras ou une jambe par exemple. Une telle prothèse comporte un appendice terminal substitutif de la partie manquante du membre amputé, muni d'une coque d'emboîtement ménageant une cavité destinée à recevoir le moignon du membre amputé. La coque, notamment obtenue par moulage d'un matériau plastique, comporte un débouché pour l'introduction du moignon.

Il est recherché, dans le domaine d'organiser la coque de manière à permettre une mise en place et un retrait du moignon qui soient simples et rapides, tout en garantissant une fiabilité de la jonction par emboîtement obtenue entre le moignon et la prothèse. A cet effet, il a été proposé d'organiser un emboîtement étanche à l'air entre le moignon et la coque pour interdire leur désolidarisation spontanée. Le moignon est préalablement enveloppé d'un manchon de forme extérieure complémentaire à la cavité de la coque, pour interdire un passage d'air entre eux et en conséquence retenir spontanément le moignon à l'intérieur de la coque. Pour autoriser la mise en place du moignon à l'intérieur de la coque, l'air résiduel contenu dans la cavité est chassé à travers un évent ménagé à la base de la coque. Cet évent est équipé d'un bouchon amovible qui est prévu pour être mis en place après l'introduction du moignon à l'intérieur de la coque en vue d'interdire une introduction d'air à l'intérieur de la cavité, et qui est prévu pour être retiré en vue d'autoriser une entrée d'air à l'intérieur de la cavité lorsque l'utilisateur souhaite enlever la prothèse. Pour garantir l'étanchéité à l'air entre le manchon et la coque, un organe d'étanchéité est placé à l'extrémité proximale de la coque. Par exemple, cet organe d'étanchéité est constitué d'un joint à lèvre qui est interposé entre la coque et le manchon en étant indifféremment fixé sur l'un de ceux-ci. Par exemple encore, cet organe d'étanchéité est constitué d'une gaine élastique enveloppant conjointement la coque et le manchon dans la zone du débouché de la coque.

Le document DE 605534 mentionne un dispositif de jonction par emboîtement étanche entre une prothèse et un manchon installé sur le moignon d'un membre amputé. Le dispositif comporte un clapet agencé en levier double monté à pivotement sur un axe central, et comprenant un premier bras d'actionnement soumis à un ressort de rappel, et un deuxième bras portant un bouchon destiné à obturer un orifice ménagé dans la coque. L'orifice est formé par un perçage de petit diamètre, qui sert en même temps pour le passage de l'air lors de la mise en place et le retrait du manchon enveloppant le moignon.

Selon le dispositif décrit dans le document DE 1795809U, la coque délimite une cavité de réception du manchon et comporte généralement à sa base un évent muni d'un bouchon manoeuvrable entre une position d'obturation et une position de libération de l'évent. Des moyens d'étanchéité sont en relation avec la coque et le manchon à l'extrémité proximale de la coque pour interdire un passage d'air entre eux, et le bouchon est équipé d'une valve d'évacuation d'air hors de la cavité et d'interdiction d'entrée d'air à l'intérieur de la cavité.

Le document DE 746781 se rapporte à un clapet à lèvres qui est monté fixe sur la coque de réception du moignon.

Le document US 2569790 mentionne un clapet à disque coulissant coopérant avec un siège fixe. En position d'ouverture, le même intervalle sert pour le passage de l'air lors de la mise en place et le retrait du moignon.

Les modalités de mise en place et de retrait de la prothèse restent néanmoins contraignantes pour l'utilisateur. Plus particulièrement, les manipulations répétées du bouchon sont incommodes et inconfortables. Il en ressort que des améliorations relatives à l'organisation de l'emboîtement étanche entre la coque et le manchon méritent d'être apportées. De telles améliorations ne doivent pas générer un inconfort de la prothèse, notamment par un accroissement significatif de son poids, et ne doivent pas induire une complexité de structure de la prothèse susceptible de générer des coûts de fabrication inopportuns.

### Objet de l'invention.

Lé but de la présente invention est de proposer un dispositif de jonction par emboîtement étanche entre une prothèse et un manchon installé sur le moignon d'un membre amputé, dont la mise en oeuvre est rapide et commode pour l'utilisateur. Il est aussi visé par la présente invention de proposer un tel dispositif de jonction dont la structure est simple et peu coûteuse à fabriquer, sans porter atteinte à sa fiabilité.

Le dispositif de jonction est caractérisé en ce que :
- la valve comporte un organe souple muni d'une ouverture qui est normalement fermée et qui s'ouvre sous l'effet d'une déformation de l'organe souple provoquée par une poussée d'air en provenance de la cavité, l'organe souple étant évidé et conformé en biseau orienté vers l'extérieur du bouchon, l'ouverture étant formée d'une fente ménagée en bout du biseau qui est normalement fermée par rapprochement spontané de ses bords et qui s'ouvre sous l'effet d'une déformation des parois en biseau de l'organe souple provoquée par une poussée d'air en provenance de la cavité,
- le bouchon est porté par une branche d'une pince dont l'autre branche est assemblée à la coque ,
- un organe de rappel est interposé entre les branches pour solliciter l'évent en position d'obturation.

Un tel agencement de la valve permet son implantation aisée dans le bouchon et son obtention à faible coût, notamment par son intégration de moulage avec le bouchon. Pour libérer et/ou obturer l'évent, l'utilisateur peut manoeuvrer l'articulation du bouchon sur la coque et en conséquence est exempté d'avoir à retirer le bouchon de la coque et d'avoir ensuite à le remettre en place.

Le bouchon est de préférence équipé d'une capsule qui obture une chambre ménagée autour de l'organe souple pour autoriser sa déformation. La capsule comporte un orifice d'évacuation d'air hors de la valve. La capsule permet notamment de protéger la valve de l'environnement extérieur, et notamment d'empêcher l'introduction de salissures à l'intérieur de la chambre.

La valve étant avantageusement incorporée au bouchon, celui-ci comporte un canal d'admission d'air à l'intérieur de l'évidement de l'organe souple. L'organe souple est placé à l'intérieur d'une cupule ménagée dans le bouchon, qui forme la chambre et qui reçoit par emboîtement la capsule à son débouché.

La pince est de préférence- assemblée à la coque de manière facilement réversible, tel que par emboîtement. Ces dispositions visent à permettre commodément un retrait occasionnel du bouchon, en vue par exemple d'une opération de maintenance tel qu'un remplacement du bouchon lorsque l'organe souple est affaibli et présente des déficiences de fonctionnement.

Selon un exemple de réalisation de la réversibilité aisée du montage de la pince sur la coque, la branche correspondante de la pince comporte un fût d'emboîtement à l'intérieur de l'évent.

L'invention a aussi pour objet une coque d'une prothèse destinée à recevoir un manchon installé sur le moignon d'un membre amputé, et une prothèse munie d'une telle coque. La coque et la prothèse de la présente invention sont principalement reconnaissables en ce que la coque est équipée d'un bouchon participant d'un dispositif de jonction comportant, prises isolément ou en combinaison, les caractéristiques qui ont été énoncées.

Les moyens d'étanchéité qui sont en relation avec le manchon et la coque pour interdire un passage d'air entre eux, sont susceptibles d'être d'une quelconque structure. Par exemple, de tels moyens d'étanchéité sont susceptibles d'être constitués d'un joint à lèvre interposé entre le manchon et la coque, ou encore d'être constitués d'une gaine étanche élastique enveloppant conjointement le manchon et la coque, notamment dans la zone du débouché de cette dernière.

### Description des figures.

La présente invention sera mieux comprise, et des détails en relevant apparaîtront, à la lecture de la description qui va être faite d'exemples de réalisation en relation avec les figures des planches annexées, dans lesquelles:
La fig.1 est un schéma en coupe axiale d'un exemple de réalisation d'une coque de prothèse, à l'intérieur de laquelle est introduit un manchon destiné à être installé sur le moignon d'un membre amputé.
Les fig.2 et fig.3 sont des schémas en coupe axiale d'un exemple de réalisation d'une coque de prothèse de la présente invention, qui est équipée d'un bouchon respectivement représenté en position d'obturation et en position de libération d'un évent ménagé à la base de la coque.
Les fig.4 et fig.5 sont des représentations respectivement de côté et de dessus d'un bouchon pour coque de prothèse.

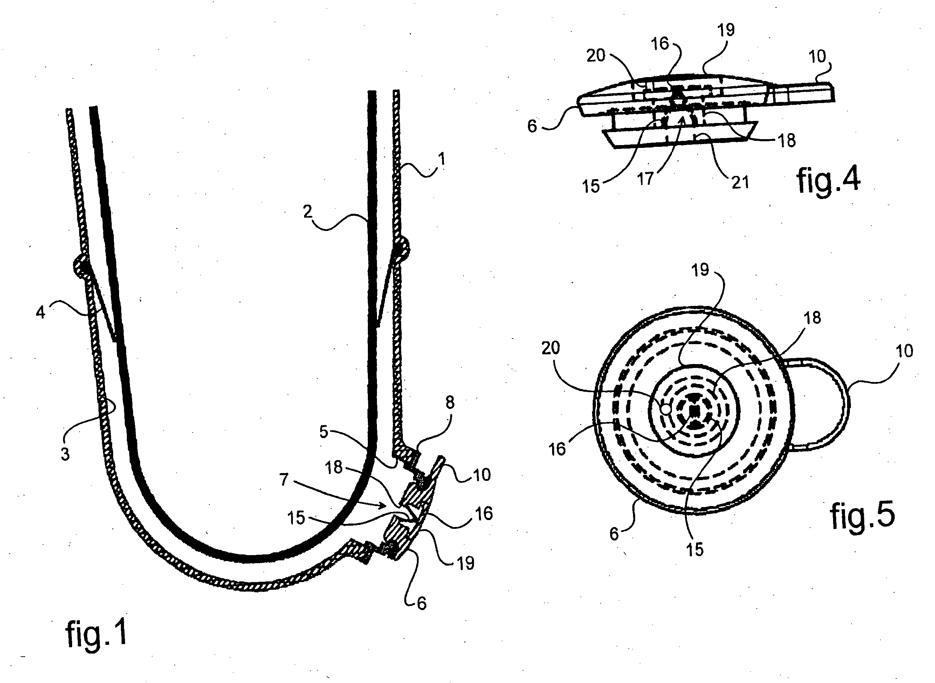

Sur la fig.1, une coque 1 de prothèse est destinée à recevoir par emboîtement étanche un manchon 2 installé sur le moignon d'un membre amputé. La conformation extérieure du manchon 2 est de forme complémentaire à celle d'une cavité 3 que comporte la coque 1 pour recevoir le manchon 2. Pour retenir la coque 1 sur le manchon 2, des moyens de type à dépression sont mis en oeuvre. Plus particulièrement, une étanchéité est organisée entre la coque 1 et le manchon 2, de sorte que le manchon 2 étant introduit à l'intérieur de la coque 1, un passage d'air vers la cavité 3 soit interdit. Cette interdiction met en oeuvre des moyens d'étanchéité 4 qui sont placés à l'extrémité proximale de la coque 1, en étant en relation avec la coque 1 et le manchon 2. Sur les exemples de réalisation illustrés, ces moyens d'étanchéité 4 sont constitués d'un joint d'interposition entre la coque 1 et le manchon 2.

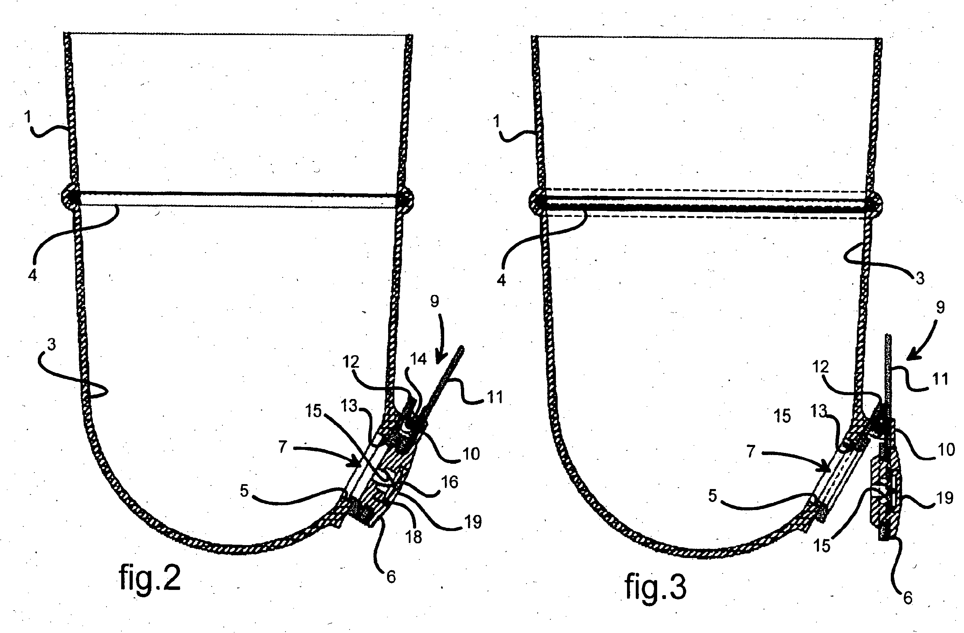

En se reportant par ailleurs aux fig.2 et fig.3, l'introduction du manchon 2 à l'intérieur de la cavité 3 est autorisée malgré la présence des moyens d'étanchéité 4, grâce à un évent 5 qui est ménagé à la base de la coque 1. Cet évent 5 est muni d'un bouchon 6, qui intègre de moulage une valve 7 de sorte que l'air chassé hors de la cavité 3, sous l'effet de l'introduction du manchon 2 à l'intérieur de la coque 1, est évacué à travers l'évent 5 puis à travers la valve 7, et de sorte qu'une introduction d'air depuis l'extérieur de la coque 1 vers la cavité 3 est interdite. Grâce à la présence de la valve 7, le bouchon 6 demeure en place sur la coque 1 entre sa position d'obturation de l'évent 5, représentée sur la fig.2, et sa position de libération de l'évent 5 représentée sur la fig.3.

Sur l'exemple de réalisation illustré sur la fis.1, le bouchon 6 est assemblé à la coque 1 par emboîtement sur un support 8, lui-même rapporté par emboîtement à l'intérieur de l'évent 5. Sur l'exemple de réalisation illustré sur les fig.2 et fig.3, le bouchon 6 est assemblé à la coque 1 par l'intermédiaire d'un support agencé en pince 9. Le bouchon 6 est avantageusement muni d'une languette de préhension 10, pour faciliter sa prise en vue de son retrait du support 8 et/ou de la pince 9. Ce retrait intervient occasionnellement, en vue d'un remplacement du bouchon 6 usé par un nouveau bouchon 6 notamment.

Plus particulièrement sur les fig.2 et fig.3, le bouchon 6 est porté par l'une des branches 11 de la pince 9, sur laquelle il est emboîté. L'autre branche 12 de la pince 9 est assemblée à la coque 1 par emboîtement, et plus particulièrement par l'intermédiaire d'un fût 13 ménagé sur cette branche 12 qui est introduit à l'intérieur de l'évent 5. Un organe de rappel 14 du bouchon 6 en position d'obturation de l'évent 5 est interposé entre les branches 11,12.

Sur l'exemple de réalisation illustré, cet organe de rappel 14 est un ressort de torsion placé autour de l'axe d'articulation des branches 11,12 entre elles. Ces dispositions permettent à l'utilisateur de manoeuvrer commodément le bouchon 6 en position de libération de l'évent 5, sans avoir à le désassembler de la coque 1, lorsqu'il souhaite retirer la prothèse.

Sur les fig.2 à fig.5, la valve 7 comporte un organe souple 15 évidé conformé en biseau orienté vers l'extérieur du bouchon 6. La pointe du biseau comporte une ouverture 16 conformée en fente, pour le passage de l'air en provenance de la cavité 3 qui circule à travers l'évidement 17 de l'organe souple 15. Cette ouverture 16 est naturellement fermée, et s'ouvre sous l'effet d'une poussée d'air chassé hors de la cavité 3 lors de l'introduction du manchon 2. La valve 7 fonctionne à partir du caractère évidé de l'organe souple 15 et de sa conformation en biseau, et à partir de la position de l'ouverture 16 en bout de biseau et de sa conformation en fente. L'air chassé hors de la cavité 3 lors de l'introduction du manchon 2 provoque son échappée à travers l'évent 5 et son passage à l'intérieur de l'évidement 17 de l'organe souple 15. La pression d'air exercée contre les faces internes de la paroi de l'organe souple 15 provoque une déformation de cette paroi et un éloignement des bords naturellement jointifs de la fente formant l'ouverture 16. En l'absence d'une telle pression d'air, voire en cas d'une éventuelle dépression à l'intérieur de la cavité 3, les bords de la fente formant l'ouverture 16 sont maintenus accolés l'un à l'autre interdisant une entrée d'air à travers l'évent 5.

Une chambre 18 est ménagée autour de l'organe souple 15 pour autoriser la déformation de ce dernier. Cette chambre 18 est conformée en cupule et est protégée par une capsule 19, qui comporte un orifice 20 autorisant l'échappée de l'air hors du bouchon 6. L'orifice 20 est ménagé de manière radialement décalée, pour ne pas interférer sur le fonctionnement de la valve 7. La capsule 19 est rapportée sur le bouchon 6 par emboîtement à l'intérieur du débouché de la chambre 18.

L'organisation de la valve 7 permet son intégration aisée de moulage avec le bouchon 6, et en conséquence un agencement du dispositif de jonction entre la coque 1 et le manchon 2 qui est de structure simple et peu coûteuse, tout en offrant un confort d'utilisation et une fiabilité idoines. L'organe souple 15 est ménagé à l'intérieur de la chambre 18 conformée en cupule, le bouchon 6 comportant un canal 21 d'admission d'air à l'intérieur de l'évidement 17 de l'organe souple 15.

## Revendications

1. Dispositif de jonction par emboîtement étanche entre une coque (1) d'une prothèse et un manchon (2) installé sur le moignon d'un membre amputé, la coque (1) délimitant une cavité (3) de réception du manchon (2) et comportant à sa base un évent (5) muni d'un bouchon (6) monté articulé sur la coque (1) sur laquelle il est maintenu entre une position d'obturation et une position de libération de l'évent (5), des moyens d'étanchéité (4) étant conjointement en relation avec la coque (1) et le manchon (2) à l'extrémité proximale de la coque (1) pour interdire un passage d'air entre eux, ledit bouchon (6) étant équipé d'une valve (7) d'évacuation d'air hors de la cavité (3) et d'interdiction d'entrée d'air à l'intérieur de la cavité (3),
**caractérisé en ce que**
- ladite valve (7) comporte un organe souple (15) muni d'une ouverture (16) qui est normalement fermée et qui s'ouvre sous l'effet d'une déformation de l'organe souple (15) provoquée par une poussée d'air en provenance de la cavité (3), l'organe souple (15) étant évidé et conformé en biseau orienté vers l'extérieur du bouchon (6), l'ouverture (16) étant formée d'une fente ménagée en bout du biseau qui est normalement fermée par rapprochement spontané de ses bords et qui s'ouvre sous l'effet d'une déformation des parois en biseau de l'organe souple (15) provoquée par une poussée d'air en provenance de la cavité (3),
- le bouchon (6) est porté par une branche (11) d'une pince (9) dont l'autre branche (12) est assemblée à la coque (1), un organe de rappel (14) étant interposé entre les branches (11,12) pour solliciter l'évent (5) en position d'obturation.

2. Dispositif de jonction selon la revendication 1, **caractérisé en ce que** le bouchon (6) est équipé d'une capsule (19) qui obture une chambre (18) ménagée autour de l'organe souple (15) pour autoriser sa déformation, la capsule (19) comportant un orifice (20) d'évacuation d'air hors de la valve (7).

3. Dispositif de jonction selon l'une quelconque des revendications précédentes, **caractérisé en ce que** la valve (7) est intégrée de moulage avec le bouchon (6).

4. Dispositif de jonction selon la revendication 3, **caractérisé en ce que** le bouchon (6) comporte un canal (21) d'admission d'air à l'intérieur de l'évidement (17) de l'organe souple (15), ce dernier étant placé à l'intérieur d'une cupule formant la chambre (18) et recevant par emboîtement la capsule (19) à son débouché.

5. Dispositif de jonction selon la revendication 4, **caractérisé en ce que** la pince (9) est assemblée à la coque (1) de manière facilement réversible.

6. Dispositif de jonction selon la revendication 4, **caractérisé en ce que** la branche (12) correspondante de la pince (9) comporte un fût (13) d'emboîtement à l'intérieur de l'évent (5).

7. Coque (1) d'une prothèse destinée à recevoir un manchon (2) installé sur le moignon d'un membre amputé, **caractérisée en ce qu'**elle est équipée d'un bouchon (6) d'un dispositif de jonction selon l'une quelconque des revendications précédentes.

## Patentansprüche

1. Vorrichtung zum Verbinden durch dichtes Ineinanderstecken zwischen einer Schale (1) einer Prothese und einer Hülse (2), die auf dem Stumpf eines amputierten Körperglieds angebracht ist, wobei die Schale (1) einen Hohlraum (3) zur Aufnahme der Hülse (2) begrenzt und an ihrer Basis ein Luftloch (5) aufweist, das mit einem Stopfen (6) versehen ist, der angelenkt an die Schale (1) montiert ist, an der er zwischen einer Verschlussposition und einer Freigabeposition des Luftlochs (5) gehalten wird, wobei Dichtungsmittel (4) gemeinsam sowohl Verbindung mit der Schale (1) als auch mit der Hülse (2) am nahen Ende der Schale (1) haben, um zwischen ihnen den Durchritt von Luft zu verhindern, welcher Stopfen (6) mit einem Ventil (7) zum Abführen der Luft aus dem Hohlraum (3) und Verhindern des Eintritts von Luft in den Hohlraum (3) versehen ist,
**dadurch gekennzeichnet, dass**
- das Ventil (7) eine elastische Vorrichtung (15) aufweist, die mit einer Öffnung (16) versehen ist, die normalerweise geschlossen ist und sich unter Wirkung einer Verformung der elastischen Vorrichtung (15) öffnet, die durch einen aus dem Hohlraum (3) kommenden Luftstoß hervorgerufen wird, wobei die elastische Vorrichtung (15) ausgehöhlt und abgeschrägt zum Äußeren des Stopfens (6) hin geformt ist und die Öffnung (16) von einem Spalt gebildet wird, der sich am Ende der Schräge befindet und normalerweise durch spontane Annäherung seiner Ränder geschlossen wird, und der sich unter Wirkung einer Verformung der schrägen Wände der elastischen Vorrichtung öffnet (15), die durch einen aus dem Hohlraum (3) kommenden Luftstoß hervorgerufen wird,
- der Stopfen (6) von einem Arm (11) einer Klammer (9) getragen wird, deren anderer Arm (12) mit der Schale (1) verbunden ist, wobei ein Rückholelement (14) zwischen den Armen (11, 12) vorgesehen ist, um das Luftloch (5) in die Verschlussstellung zu ziehen.

2. Verbindungsvorrichtung nach Anspruch 1, **dadurch gekennzeichnet, dass** der Stopfen (6) mit einer Kapsel (19) versehen ist, die eine Kammer (18) verschließt, die um die elastische Vorrichtung (15) herum angeordnet ist, um deren Verformung zu erlauben, wobei die Kapsel (19) eine Öffnung (20) zum Abführen von Luft aus dem Ventil (7) aufweist.

3. Verbindungsvorrichtung nach einem der vorstehenden Ansprüche, **dadurch gekennzeichnet, dass** das Ventil (7) fest an den Stopfen (6) geformt ist.

4. Verbindungsvorrichtung nach Anspruch 3, **dadurch gekennzeichnet, dass** der Stopfen (6) einen Kanal (21) zur Luftzufuhr in das Innere der Aussparung (17) der elastischen Vorrichtung (15) aufweist, wobei dieser innerhalb einer Kupula angeordnet ist, die die Kammer (18) bildet und durch Ineinanderstecken die Kapsel (19) an ihrer Mündung aufnimmt.

5. Verbindungsvorrichtung nach Anspruch 4, **dadurch gekennzeichnet, dass** die Klammer (9) mit der Schale (1) so verbunden ist, dass sie problemlos umkehrbar ist.

6. Verbindungsvorrichtung nach Anspruch 4, **dadurch gekennzeichnet, dass** der entsprechende Arm (12) der Klammer (9) einen Schaft (13) zum Einstecken in das Luftloch (5) aufweist.

7. Schale (1) einer Prothese, die vorgesehen ist, eine Hülse (2) aufzunehmen, die auf dem Stumpf eines amputierten Körperglieds angebracht ist, **dadurch gekennzeichnet, dass** sie mit einem Stopfen (6) einer Verbindungsvorrichtung nach einem der vorstehenden Ansprüche versehen ist.

## Claims

1. Joining device by watertight fitting between a shell (1) of a prosthesis and a sleeve (2) installed on the stump of an amputated member, the shell (1) delineating a cavity (3) receiving the sleeve (2) and comprising at its base a vent (5) provided with a plug (6) mounted articulated on the shell (1) on which it is secured between a sealing position and a releasing position of the vent (5), sealing means (4) being jointly in relation with the shell (1) and the sleeve (2) at the proximal end of the shell (1) to prevent passage of air between the two, said plug (6) being equipped with a valve (7) for removing air from the cavity (3) and for preventing air from getting inside the cavity (3), **characterized in that**
- said valve (7) comprises a flexible part (15) provided with an opening (16) which is normally closed and which opens due to the effect of a deformation of the flexible part (15) caused by a thrust of air originating from the cavity (3), the flexible part (15) being hollowed and in the form of a bevel directed towards the outside of the plug (6), the opening (16) being formed by a slot arranged at the end of the bevel which is normally closed by spontaneous movement of its edges towards one another and which opens due to the effect of a deformation of the bevelled walls of the flexible part (15) caused by a thrust of air originating from the cavity (3),
- the plug (6) is supported by a branch (11) of a grip (9) the other branch (12) of which is assembled to the shell (1), a bias part (14) being fitted between the branches (11,12) to bias the vent (5) to the sealed position.

2. Joining device according to claim 1, **characterized in that** the plug (6) is equipped with a capsule (19) which tightly seats a chamber (18) arranged around the flexible part (15) to allow deformation of the latter, the capsule (19) comprising an opening (20) for removing the air from the valve (7).

3. Joining device according to any one of the foregoing claims, **characterized in that** the valve (7) is integrated by moulding with the plug (6).

4. Joining device according to claim 3, **characterized in that** the plug (6) comprises a channel (21) for inlet of air to the inside of the recess (17) of the flexible part (15), the latter being placed inside a cupule forming the chamber (18) and receiving the capsule (19) by interlocking at its outlet.

5. Joining device according to claim 4, **characterized in that** the grip (9) is assembled to the shell (1) in easily reversible manner.

6. Joining device according to claim 4, **characterized in that** the corresponding branch (12) of the grip (9) comprises an engagement shank (13) inside the vent (5).

7. Shell (1) of a prosthesis designed to receive a sleeve (2) installed on the stump of an amputated member, **characterized in that** it is equipped with a plug (6) of a joining device according to any one of the foregoing claims.
